# EUROPEAN PATENT APPLICATION

(11) **EP 4 817 727 A1**
(43) Date of publication of application: **07.10.2026**
(21) Application number: 25168659.8
(22) Date of filing: 04.04.2025
(51) Int. Cl.: B01F 31/23, B01F 35/22, B01F 35/92, B01J 19/28, C12M 3/06, B01F 35/90

(54) **ROCKING DEVICE FOR A BIOREACTOR**

(71) Applicant: Sartorius Stedim Biotech GmbH, 37079 Göttingen (DE)
(72) Inventor: Heuer, Dennis, 37079 Göttingen (DE); Wiegand, Jan, 34302 Guxhagen (DE); Topp-Manske, Simon, 34302 Guxhagen (DE); Wolff, Alexander, 37079 Göttingen (DE); Schenk, Tobias, 37079 Göttingen (DE); Knabe, Rene, 37079 Göttingen (DE); Hartmann, Felix, 37079 Göttingen (DE)
(74) Representative: Gottschald Patentanwälte Partnerschaft mbB

(57) **Abstract**

The invention relates to a rocking device for a bioreactor (2), wherein the rocking device (1) comprises a base (3) and a platform (4) carried by the base (3), wherein the rocking device (1) comprises a rocking mechanism (5) comprising a motor (6), wherein the rocking device (1) comprises a control unit (7), wherein the control unit (7) is configured to cause the motor (6) to rock the platform (4) relative to the base (3) around an axis (8), wherein the platform (4) comprises a first end (9) and a second end (10) located on opposite sides of the axis (8), wherein the rocking mechanism (5) is configured to tilt the platform (4) around the axis (8) starting from a horizontal position in a first direction (11) to a first maximum angle and in an opposite second direction (12) to a second maximum angle. It is proposed that the control unit (7) is configured to perform an asymmetric rocking routine comprising at least one asymmetric rocking motion, and, that the asymmetric rocking motion in the asymmetric rocking program comprises tilting the platform (4) in the first direction (11) to a first angle (α), and, tilting the platform (4) in the second direction (12) to a second angle (β) different from the first angle (α).

## Description

The present invention relates to a rocking device for a bioreactor according to the general part of claim 1, to a rocking arrangement comprising a rocking device for a bioreactor according to the general part of claim 15 and to a method of controlling a rocking arrangement according to claim 17.

Bioreactors in bioprocessing often utilize rocking devices to provide controlled mixing, aeration, and nutrient distribution. These devices sometimes offer not only motion control but also temperature regulation, including heating and cooling capabilities. The specific requirements for motion profiles and temperature profiles vary depending on the bioprocess. Additionally, batch-to-batch variations in mixing profiles can significantly impact the bioprocess and, ultimately, the resulting bioproduct. Therefore, good repeatability of mixing is desired.

A single rocking device is frequently used for multiple bioprocesses. Using the same device for different bioprocesses also helps users adapt more easily to new processes without the need to learn a new system. Consequently, a highly versatile rocking device is desirable.

Known rocking devices are sometimes optimized for a small set of use cases or usable for most use cases without specific optimization. Many rocking devices provide symmetric motion profiles, which covers usual use cases. However, a more versatile rocking device that still covers usual use cases is desired.

In detail, the present teaching is based on improving a rocking device for a bioreactor, wherein the rocking device comprises a base and a platform carried by the base, wherein the rocking device comprises a rocking mechanism comprising a motor, wherein the rocking device comprises a control unit, wherein the control unit is configured to cause the motor to rock the platform relative to the base around an axis, wherein the platform comprises a first end and a second end located on opposite sides of the axis, wherein the rocking mechanism is configured to tilt the platform around the axis starting from a horizontal position in a first direction to a first maximum angle and in an opposite second direction to a second maximum angle. The rocking device is therefore a rocking device of the seesaw type. The mechanical design of such a rocking device is well usable for symmetric rocking.

The invention is based on the problem of improving the known rocking devices by providing coverage for other, in particular additional, use cases. It is desirable not to increase the mechanical complexity and therefore price of the rocking device.

The above-noted object is solved by the features of the characterizing part of claim 1.

The main realization of the present invention is that a rocking device of the described "seesaw" type can be designed such that asymmetric motions become possible through adequate software in the control unit. Enabling asymmetric motions provides increased coverage for use cases such as in vitro transcription, microorganism based bioprocesses, cell-free bioprocesses, cell and gene therapy bioprocesses and the like, in particular bioprocesses with a small liquid volume compared to the capacity of the rocking device.

In detail, it is proposed that the control unit is configured to perform an asymmetric rocking routine comprising at least one asymmetric rocking motion, and, that the asymmetric rocking motion in the asymmetric rocking program comprises tilting the platform in the first direction to a first angle, and, tilting the platform in the second direction to a second angle different from the first angle.

Claim 2 describes two preferred embodiments. In the first embodiment, the rocking motion is performed only on one side of the horizontal position. A bioreactor lying flat on the platform is then rocked without returning to its horizontal position. That is especially helpful during a media removal routine. Conventional rocking motion "from side to side" may lead to a port like a harvest port or other liquid contacting element like a filter or sensor not being in contact with the liquid. Conventionally, for harvesting, sampling and the like, the rocking motion is stopped. However, that can lead to cells or other particles in the liquid settling, which in turn leads to problems with sensor measurements, for example. By rocking only on the side with the port, the liquid is kept mixing during removal of some or all of the content (media) of the bioreactor.

In the second embodiment, the horizontal position is traversed during the rocking motion. That may improve the mixing but still allows for new wave forms, adapting to asymmetric bags and the like.

In a preferred embodiment according to claim 3, the rocking device is also configured to perform a symmetric rocking motion. It may still be the case that many or most use cases require symmetric rocking. However, a symmetric rocking motion device with asymmetric rocking capabilities provides an increased versatility.

The rocking device may comprise a tilt sensor for detecting an absolute horizontal position (claim 4). With a tilt sensor, several features can be implemented.

For example, a mechanical levelling mechanism present in an embodiment according to claim 5 may be supported by displaying a tilt on a display digitally imitating a spirit level.

A tilt sensor also allows, at least to some degree, for correcting the horizontal position of the platform if the platform is not well levelled (claim 6). For example, a programmed rocking from -10° to +10° may be performed from -9° to +11° if the rocking device is placed on a ground with a 1° slope and not manually levelled.

Claims 7 and 8 describe embodiments relating to the rocking mechanism which are well suited for implementing the symmetric and asymmetric rocking motion and other preferred embodiments.

Claim 9 to 14 describe preferred embodiments related to heating and/or cooling the bioreactor. It is noted that these embodiments are seen as relevant independent of the asymmetric rocking motion, too.

Therefore, a rocking device for a bioreactor, wherein the rocking device comprises a base and a platform carried by the base, wherein the rocking device comprises a rocking mechanism comprising a motor, wherein the rocking device comprises a control unit, wherein the control unit is configured to cause the motor to rock the platform relative to the base around an axis, characterized in that the rocking device comprises a heating arrangement for heating and/or cooling the bioreactor, is also proposed herein. Reference is made to all features described with regard to the proposed rocking device which may be added to this rocking device in any combination, and in particular to the features of the heating arrangement, all of which are relevant on their own.

Claim 9 describes embodiments with preferably two electrical heating zones, allowing for a better control of the temperature of the bioreactor, specifically also for small bioreactors. Claim 10 further details preferred embodiments of the zones and their control. In particular, the second heating zone may be located outwards of the first heating zone, the heating zones thereby supporting smaller and bigger bioreactors by using either only the first heating zone or both zones. The second heating zone may comprise two sub-zones controlled together to assure symmetric heating.

In an embodiment according to claim 11, the control unit detects a property of the bioreactor and controls the zones accordingly. Claim 12 relates to the control of a contact temperature for the bioreactor. It is important to protect the content of the bioreactor from overheating. So far, this involves conservative heating strategies and manual stopping of the heating during a standstill (no rocking). It is problematic if a bioreactor is heated while not rocking, especially if the bioreactor comprises a bag which may not be fully expanded, or when handling small volumes. High contact temperatures may damage the cells, organisms, proteins or the like inside the bioreactor. It is proposed as one embodiment to provide a temperature sensor and control the heating arrangement, in particular to limit the contact temperature. This limiting of the contact temperature protects the content of the bioreactor during rocking and during a standstill.

Another embodiment is described in claim 13. In this embodiment, a heat-transfer fluid is used for heating and/or cooling. The rocking device may comprise a weight sensor or weight sensors. The accuracy of the weight sensors can be increased by using the tilt sensor and/or by increasing the precision of the rocking with the described preferred rocking mechanism. Of particular interest is the embodiment according to claim 14, in which a pillow plate is used as the heat transfer element. A pillow plate provides interesting fluid dynamics during rocking, significantly reducing unbalances created by the movement of the heat-transfer fluid as the heat-transfer fluid is inhibited from forming a wave from the rocking. Inhibiting this wave also increases the precision of the weight sensors and possibly the tilt sensor, enabling a more precise weighing and control of the rocking device.

Another teaching according to claim 15, which is of equal importance, relates to a rocking arrangement comprising a rocking device for a bioreactor.

Here, it is proposed that the control unit is configured to perform an asymmetric rocking routine comprising at least one asymmetric rocking motion, and, that the asymmetric rocking motion in the asymmetric rocking program comprises tilting the platform in the first direction to a first angle, and, tilting the platform in the second direction to a second angle different from the first angle, preferably, that the control arrangement comprises an internal control unit, in particular as the control unit, as part of the rocking device, and/or, that the control arrangement comprises an external control unit.

All explanations given with regard to the proposed rocking device are fully applicable.

Claim 16 describes a possible temperature boost for cooling or heating created by stopping the supply of the heat-transfer fluid, cooling or heating the fluid, and then resuming the supply.

This embodiment is also relevant independent of the asymmetric motion, such that a rocking arrangement comprising a rocking device for a bioreactor, wherein the rocking device comprises a base and a platform carried by the base, wherein the rocking device comprises a rocking mechanism comprising a motor, wherein the rocking arrangement comprises a control arrangement comprising at least one control unit, wherein the control unit is configured to cause the motor to rock the platform relative to the base around an axis, characterized in that the external control unit or the rocking device comprises a fluid source, in particular a fluid tank, connected to the feed arrangement providing the heat-transfer fluid to the heat transfer element, and a heating and/or cooling device for heating and/or cooling the heat-transfer fluid, and, that the external control unit is configured to, in a boost routine, stop the supply of the heat-transfer fluid to the heat transfer element, increase or decrease the temperature of the heat-transfer fluid, in particular in the tank, and restart supplying the heat-transfer fluid to the heat-transfer element, is also proposed herein. Reference is made to all features described with regard to the proposed rocking device and arrangement which may be added to this rocking device and arrangement in any combination.

Another teaching according to claim 17, which is of equal importance, relates to a method of controlling a proposed rocking arrangement.

All explanations given with regard to the proposed rocking device and the proposed rocking arrangement are fully applicable. In particular, any steps the rocking device or any part of it, in particular the control unit, is described as being configured to perform may be performed as part of the method. For example, the control unit may perform the asymmetric rocking routine as part of the method.

In the following, embodiments of the invention are explained with respect to the drawing. The drawing shows in
- Fig. 1: a) a rocking arrangement comprising a rocking device, b) an asymmetric rocking and c) a symmetric rocking,
- Fig. 2: a view onto the platform of the rocking device and the heating zones and
- Fig. 3: an alternative or additional heating embodiment using a pillow plate.

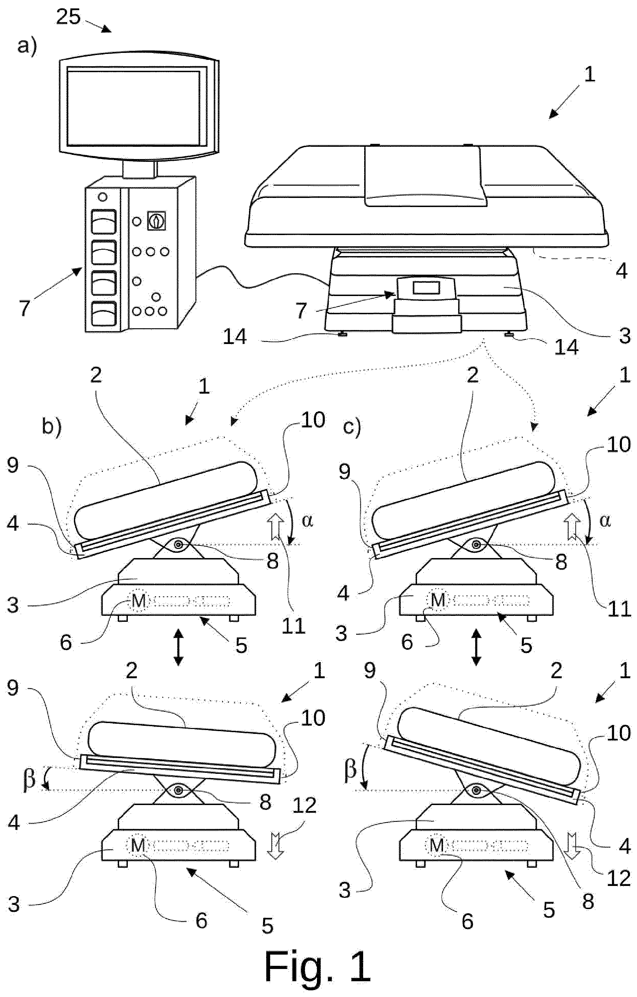

Proposed and shown in the figures is a rocking device 1 for a bioreactor 2. The bioreactor 2 preferably comprises a bioreactor bag. The rocking device 1 comprises a designated space for the bioreactor 2 and generally provides a rocking motion to the bioreactor 2 for contactless mixing of a liquid inside the bioreactor 2. Fig. 1 a) shows the proposed rocking device 1 on the right side. The bioreactor 2 may have an inner volume of for example at least 100 L or at least 200 L.

The rocking device 1 comprises a base 3 and a platform 4 carried by the base 3. The rocking device 1 also comprises a rocking mechanism 5 comprising a motor 6. The motor 6 is shown only schematically, however, several ways for rocking the platform 4 are known. The motor 6 is connected to the platform 4 such that the motor 6 may rock the platform 4.

The rocking device 1 comprises a control unit 7, wherein the control unit 7 is configured to cause the motor 6 to rock the platform 4 relative to the base 3 around an axis 8. The axis 8 here and preferably is an axis 8 parallel to the platform 4 and/or horizontally oriented, in the figures it protrudes into the plane of the drawing.

The platform 4 comprises a first end 9 and a second end 10 located on opposite sides of the axis 8. The platform 4 therefore moves like a seesaw around the axis 8. "Rocking" can therefore be described as a tilting back and forth around the axis 8. Preferably, the platform 4 is configured to be tilted only around the axis 8. The axis 8 may be formed by a hinge. The axis 8 may be below the center of the platform 4 such that both ends are placed at an equal distance from the axis 8. Preferably, the axis 8 is located at least in the center 20 % of the length of the platform 4 between the ends.

The rocking mechanism 5 is configured to tilt the platform 4 around the axis 8 starting from a horizontal position in a first direction 11 to a first maximum angle and in an opposite second direction 12 to a second maximum angle. The first maximum angle and the second maximum angle may be defined by the mechanical features of the rocking mechanism 5 and/or may be equal (i.e. have the same size in opposite directions) at least when the horizontal position is equal to an absolute horizontal position. In the figures, the first direction 11 and the second direction 12 correspond to clockwise or counter-clockwise movements.

Proposed is that the control unit 7 is configured to perform an asymmetric rocking routine comprising at least one asymmetric rocking motion, and, that the asymmetric rocking motion in the asymmetric rocking program comprises tilting the platform 4 in the first direction 11 to a first angle α, and, tilting the platform 4 in the second direction 12 to a second angle β different from the first angle α. Fig. 1 b) shows the platform 4 tilted into the first angle α at the top and into the second angle β at the bottom. The asymmetric rocking motion then comprises at least one movement between these positions. It should be noted that the first angle α and the second angle β describe the end position of a movement in the respective direction, i.e. the position at which the tilting direction is reversed. The second angle β is preferably smaller than the first angle α. The difference between the first angle α and the second angle β is preferably at least 1°, more preferably at least 2°, more preferably at least 3°, more preferably at least 4°, more preferably at least 5°, more preferably at least 6°,more preferably at least 7°,more preferably at least 8°. The tilting may smoothly reverse at the first angle α and/or second angle β or stop at the respective angle and/or at the horizontal position before resuming the movement.

Preferably, the rocking device 1 is configured to provide up to 60 rounds of movements in the first direction 11 and the second direction 12 per minute. The first maximum angle and/or the second maximum angle may be at least 13°, preferably at least 15°, more preferably at least 17°, and/or at most 45°, preferably at most 30°, more preferably at most 20°. The rocking device 1 may also comprise a display for displaying for example the parameter of the rocking motion and/or an interface for receiving parameters of the rocking. Preferably, the user may input the first angle α and the second angle β, in particular in any combination within the possible ranges.

It is possible that the second angle β is a negative angle such that the rocking motion is performed on one side of the horizontal position. In this case, the platform 4 does not tilt into and therefore also not through the horizontal position. This embodiment can be used for several use cases. In a preferred use case, the control unit 7 is configured to perform the rocking motion during a media removal routine. The control unit 7 may even control the media removal or may otherwise be put into the dedicated media removal routine. Alternatively, the user may simply configure the control unit 7 to perform the rocking motion without starting a dedicated media removal routine. During the media removal routine, a content of the bioreactor 2 is harvested or a part of the content is sampled or removed for other reasons. The bioreactor 2 may comprise a port, in particular harvest port, on one side and the rocking may be done only on that side to always keep the port in contact with the liquid. At the same time, due to the rocking during media removal, sensor measurements, for example biomass sensor measurements, may be more precise. It is preferably the case that the draining of the liquid in the bioreactor 2 during the media removal is not stopped during the rocking motion, though that is optional.

Alternatively and as shown in Fig. 1 b), the second angle β is a positive angle such that the rocking motion is performed on both sides of the horizontal position and such that the rocking motion is performed tilting through the horizontal position. It is also preferred that the control unit 7 is configured to perform two asymmetric rocking routines, one in which the second angle β is positive and one in which the second angle β is negative. If the user can choose any possible combination of angles, that follows as a consequence.

It can already be derived that the rocking device 1 is also configured to provide symmetric rocking. Here and preferably, the control unit 7 is configured to perform a symmetric rocking routine comprising at least one symmetric rocking motion. The symmetric rocking motion in the symmetric rocking program comprises tilting the platform 4 from the horizontal position in the first direction 11 to a first angle α, reversing the platform 4 to the horizontal position, and, tilting the platform 4 from the horizontal position in the second direction 12 to a second angle β, equal to the first angle α. The symmetric rocking is shown in Fig. 1 c).

It can also be said for any of the rocking routines that preferably the respective rocking motion is repeated at least 10 times, preferably at least 100 times, or at least for 5 minutes or for any time set by the user or until stopped by the user. Preferably, the repetitions are all equal, though more complex movements with changing first angles α and second angles β are also conceivable.

The rocking device 1 may comprise at least one tilt sensor 13, in particular an accelerometer and/or gyroscope, for measuring the tilt of the platform 4 and/or base 3 relative to an absolute horizontal position. The horizontal position on the other hand may be a relative horizontal position defined in the control unit 7. For a number of reasons, the horizontal position of the platform 4 and/or base 3 may deviate from the "real" absolute horizontal position. For example, a floor or desk on which the rocking device 1 is placed may be slightly inclined.

Accordingly, it may be the case that the rocking device 1 comprises a mechanical levelling mechanism 14 for levelling the platform 4 and/or base 3. For example, the mechanical levelling mechanism 14 may comprise adjustable feet connected to the base 3 for placing the base 3 on a ground. The length of the feet may be adjustable to tilt the rocking device 1 into the correct position.

As mentioned, the rocking device 1 may comprise a display. On this display, the control unit 7 may display, in a mechanical leveling routine, an indication of a difference between the relative horizontal position and the absolute horizontal position. This difference can be derived by the control unit 7 via the tilt sensor 13. In this manner, the user is assisted in levelling the platform 4. The control unit 7 may also document changes in the levelling of the platform 4 to form an audit trail.

Alternatively or if the mechanical levelling is not precise enough or the levelling has changed afterwards, it may be the case that the control unit 7 redefines, in an electronic leveling routine, the relative horizontal position to reduce a difference between the relative horizontal position and the absolute horizontal position. Preferably however, the control unit 7 does not perform the electronic leveling routine automatically while performing any rocking program. A deviation of for example up to 2° or more may be corrected by the control unit 7. The electronic leveling routine may be triggered by a user or automatically, for example after the mechanical levelling routine. During rocking the rocking is preferably not influenced by a change of the horizontal position.

Turning towards the rocking mechanism 5, to provide for a precise asymmetric and preferably symmetric rocking, the motor 6 may be a stepper motor or a servo motor and/or the control unit 7 may be configured to control the motor 6 using a, in particular closed, control loop with a position of the motor 6, in particular a position of a rotor of the motor 6, as a controlled parameter. It may also be the case that the motor 6 comprises an integrated position sensor. The integrated position sensor is preferably an absolute position sensor.

Fig. 1 shows in b) and c) schematically that the rocking mechanism 5 may comprise a spindle 15 transforming rotatory movements of the motor 6 into linear movements. Here and preferably, the rocking mechanism 5 also comprises a sled 16 connected between the spindle 15 and the platform 4. The sled 16 may then act on the platform 4 directly or indirectly. Preferably, a lever (not shown) is connected between the sled and the platform 4.

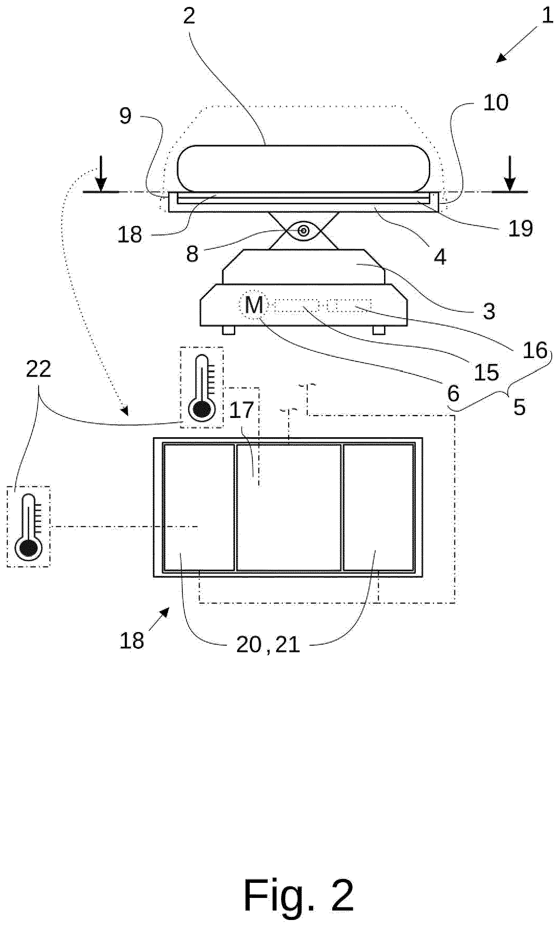

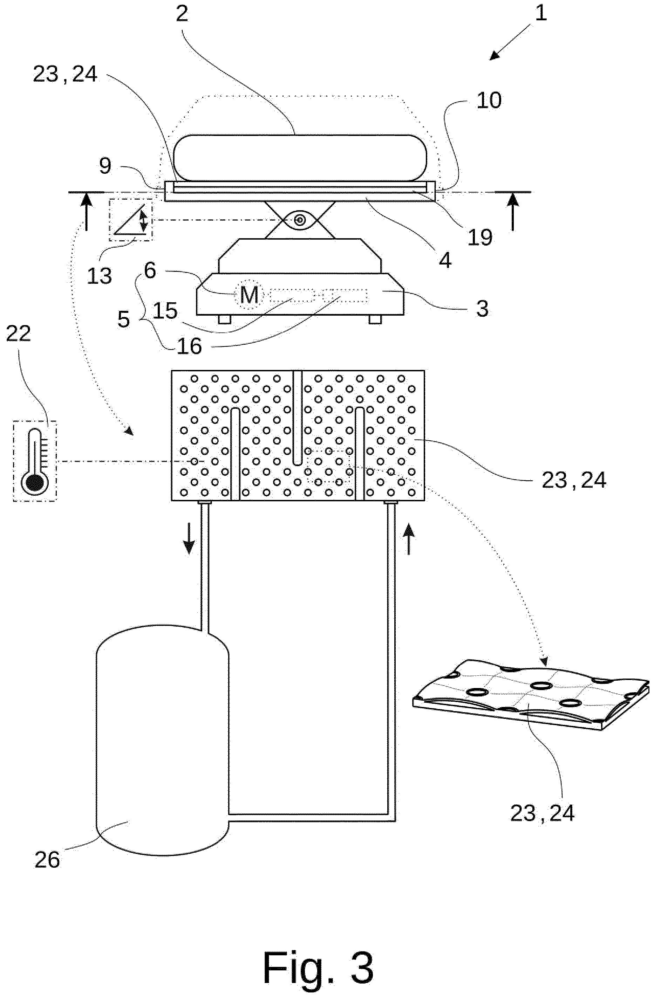

Turning towards figures 2 and 3, starting with Fig. 2, it is here and preferably the case that the rocking device 1 comprises a heating arrangement for heating and/or cooling the bioreactor 2.

Preferably, the heating arrangement comprises a, in particular electrical, first heating zone 17 located on the platform 4, in particular below the bioreactor 2. Fig. 2 shows a view onto the platform 4 and in particular an electrical heating element 18 of the platform 4 comprising the first heating zone 17. Below the electrical heating element 18, an insulating layer 19 is shown.

It is here and preferably the case that the heating arrangement comprises a, in particular electrical, second heating zone 20 located on the platform 4, in particular below the bioreactor 2. The first heating zone 17 and preferably the second heating zone 20 are preferably located such that they are rocked with the bioreactor 2, allowing for heating during the rocking.

It is preferred that the control unit 7 is configured to control the first heating zone 17 and the second heating zone 20 differently. As Fig. 2 shows, in one embodiment, the second heating zone 20 comprises two sub-zones 21 located at two sides of the first heating zone 17, in particular one located towards the first end 9 and one located towards the second end 10. This arrangement allows heating smaller and larger bioreactors 2 differently. The two sub-zones 21 are preferably controlled by the control unit 7 together and/or are located at a distance from each other, as shown. The sub-zones 21 may each have a length of at least 10 % of the length between the first end 9 and the second end 10 along a direction between the first end 9 and the second end 10. Alternatively, the first heating zone 17 and the second heating zone 20 may be adjacent to each other and the rocking device 1 may be configured to rock and heat two bioreactors 2, each placed on one of the heating zones 17, 20. Each heating zones 17, 20 may be located on one half of the platform 4.

Preferably, the control unit 7 is configured to detect a property, in particular a position and/or a size, of the bioreactor 2 and control the first heating zone 17 and/or the second heating zone 20 based on the detection. Automatically adjusting the heating is convenient for a user and reduces possible mistakes by the user in programming the heating. Preferably, the control unit 7 is configured to detect via at least one sensor, in particular the tilt sensor 13 and/or at least one weight sensor, a mixing characteristic of the bioreactor 2 associated with the property of the bioreactor 2. This is particularly useful if only a few bioreactors 2 are used with the rocking device 1 and known to the control unit 7.

According to one embodiment it is proposed that the heating arrangement comprises a temperature sensor 22, in particular contact temperature sensor, and that the control unit 7 is configured to control the heating arrangement based on measurements from the temperature sensor 22. The control unit 7 may prevent the contact temperature from rising above a predefined, in particular user-defined, limit temperature. Different from a temperature control based on introducing a certain amount of heat, limiting the contact temperature helps avoiding hot spots which may damage the content of the bioreactor 2.

Preferably, the control unit 7 is configured to control the heating arrangement using a, in particular closed, control loop with the temperature, in particular contact temperature, as a controlled parameter, in particular during a standstill of the platform 4. When the platform 4 is not rocking, the danger of damaging the content of the bioreactor 2 increases. The limit temperature may be reduced during a standstill.

Turning towards Fig. 3, in one embodiment it is proposed that the heating arrangement comprises a heat-transfer fluid arrangement for heating and/or cooling the bioreactor 2, and, that the heat-transfer fluid arrangement comprises a heat transfer element 23 located on the platform 4, in particular below the bioreactor 2, and a feed arrangement for feeding the heat-transfer fluid to the heat transfer element 23. As an alternative to electrical heating or conceivably even in addition thereto, a heat-transfer fluid can be used. The feed arrangement may be fed from a source external to the rocking device 1 to the rocking device 1.

It is preferably the case and shown in Fig. 3, that the heat transfer element 23 is a, in particular single-embossed, pillow plate 24. The heat-transfer fluid is then introduced into the pillow plate 24. On the side towards the bioreactor 2 the pillow plate 24 may have a plane side while the other side may comprise embossings. The heat-transfer fluid flows between the embossings and heats the sides of the pillow plate 24. Due to the embossings, the heat-transfer fluid does not or only to a small extent from a wave during rocking. That helps for example with the detection of the bioreactor 2 and increases the precision of tilt and weight measurements.

Another teaching which is of equal importance relates to a rocking arrangement 25 comprising a rocking device 1 for a bioreactor 2, wherein the rocking device 1 comprises a base 3 and a platform 4 carried by the base 3, wherein the rocking device 1 comprises a rocking mechanism 5 comprising a motor 6, wherein the rocking arrangement 25 comprises a control arrangement comprising at least one control unit 7, wherein the control unit 7 is configured to cause the motor 6 to rock the platform 4 relative to the base 3 around an axis 8, wherein the platform 4 comprises a first end 9 and a second end 10 located on opposite sides of the axis 8, wherein the rocking mechanism 5 is configured to tilt the platform 4 around the axis 8 starting from a horizontal position in a first direction 11 to a first maximum angle and in an opposite second direction 12 to a second maximum angle. The control unit 7 described for the rocking device 1 may be external to the rocking device 1 as part of the rocking arrangement 25.

Proposed according to this further teaching is that the control unit 7 is configured to perform an asymmetric rocking routine comprising at least one asymmetric rocking motion, and, that the asymmetric rocking motion in the asymmetric rocking program comprises tilting the platform 4 in the first direction 11 to a first angle α, and, tilting the platform 4 in the second direction 12 to a second angle β different from the first angle α, preferably, that the control arrangement comprises an internal control unit 7, in particular as the control unit 7, as part of the rocking device 1, and/or, that the control arrangement comprises an external control unit 7. All steps, routines and the like described for the internal control unit 7 may be performed partially or fully by the external control unit 7.

It is preferably the case that the external control unit 7 or the rocking device 1 comprises a fluid source, in particular a fluid tank 26, connected to the feed arrangement providing the heat-transfer fluid to the heat transfer element 23, and a heating and/or cooling device for heating and/or cooling the heat-transfer fluid.

In a preferred embodiment, the external control unit 7 is configured to, in a boost routine, stop the supply of the heat-transfer fluid to the heat transfer element 23, increase or decrease the temperature of the heat-transfer fluid, in particular in the tank, and restart supplying the heat-transfer fluid to the heat-transfer element. The external control unit 7 may be configured to restart supplying the heat-transfer fluid when a predetermined temperature, in particular contact temperature, is reached. The predetermined temperature may be a maximum or minimum allowed temperature defined in the external control unit 7. In this manner, a maximum temperature difference between the bioreactor 2 and the heat transfer fluid can be reached, thereby generating a strong heat or cold shock. Some bioprocesses require a heat shock, for example to deactivate enzymes, or a quick cooling to increase the quality of the bioproduct. This boost routine provides exactly that. Due to the slow change in temperature of the heat-transfer fluid in the heat transfer element 23, stopping the supply for some time is usually not problematic. Then, the newly added heat-transfer fluid has a substantially different temperature, providing said temperature boost. If the supply wasn't stopped, the change in temperature could be done only at a rate limited by the capability of the external control unit 7.

A maximum temperature that the heat-transfer fluid can be heated to by the rocking arrangement 25 is preferably at least 40° C, more preferably at least 50° C, more preferably at least 60° C, more preferably at least 70° C, more preferably at least 80° C. A minimum temperature that the heat-transfer fluid can be cooled to by the rocking arrangement 25 is preferably at most 20° C, more preferably at most 10° C, more preferably at most 5° C.

Another teaching which is of equal importance relates to a method of controlling a proposed rocking arrangement 25.

## Claims

1. Rocking device for a bioreactor (2), wherein the rocking device (1) comprises a base (3) and a platform (4) carried by the base (3), wherein the rocking device (1) comprises a rocking mechanism (5) comprising a motor (6), wherein the rocking device (1) comprises a control unit (7), wherein the control unit (7) is configured to cause the motor (6) to rock the platform (4) relative to the base (3) around an axis (8),
wherein the platform (4) comprises a first end (9) and a second end (10) located on opposite sides of the axis (8), wherein the rocking mechanism (5) is configured to tilt the platform (4) around the axis (8) starting from a horizontal position in a first direction (11) to a first maximum angle and in an opposite second direction (12) to a second maximum angle,
**characterized in**
**that** the control unit (7) is configured to perform an asymmetric rocking routine comprising at least one asymmetric rocking motion, and, that the asymmetric rocking motion in the asymmetric rocking program comprises tilting the platform (4) in the first direction (11) to a first angle (α), and, tilting the platform (4) in the second direction (12) to a second angle (β) different from the first angle (α).

2. Rocking device according to claim 1, **characterized in that** the second angle (β) is a negative angle such that the rocking motion is performed on one side of the horizontal position, preferably, that control unit (7) is configured to perform the rocking motion during a media removal routine,
or, that the second angle (β) is a positive angle such that the rocking motion is performed on both sides of the horizontal position and tilting through the horizontal position.

3. Rocking device according to claim 1 or 2, **characterized in that** the control unit (7) is configured to perform a symmetric rocking routine comprising at least one symmetric rocking motion, and, that the symmetric rocking motion in the symmetric rocking program comprises tilting the platform (4) from the horizontal position in the first direction (11) to a first angle (α), reversing the platform (4) to the horizontal position, and, tilting the platform (4) from the horizontal position in the second direction (12) to a second angle (β), equal to the first angle (α).

4. Rocking device according to one of the preceding claims, **characterized in that** the rocking device (1) comprises at least one tilt sensor (13), in particular an accelerometer and/or gyroscope, for measuring the tilt of the platform (4) and/or base (3) relative to an absolute horizontal position, and/or, that the horizontal position is a relative horizontal position defined in the control unit (7).

5. Rocking device according to one of the preceding claims, **characterized in that** the rocking device (1) comprises a mechanical levelling mechanism (14) for levelling the platform (4) and/or base (3), preferably, that the mechanical levelling mechanism (14) comprises adjustable feet connected to the base (3) for placing the base (3) on a ground, and/or, that the rocking device (1) comprises a display, and, that the control unit (7) displays, in a mechanical leveling routine, on the display an indication of a difference between the relative horizontal position and the absolute horizontal position.

6. Rocking device according to one of claims 4 or 5, **characterized in that** the control unit (7) redefines, in an electronic leveling routine, the relative horizontal position to reduce a difference between the relative horizontal position and the absolute horizontal position, preferably, that the control unit (7) does not perform the electronic leveling routine automatically while performing any rocking program.

7. Rocking device according to one of the preceding claims, **characterized in that** the motor (6) is a stepper motor or a servo motor, and/or, that the control unit (7) is configured to control the motor (6) using a, in particular closed, control loop with a position of the motor (6), in particular a position of the rotor, as a controlled parameter, preferably, that the motor (6) comprises an integrated position sensor.

8. Rocking device according to one of the preceding claims, **characterized in that** the rocking mechanism (5) comprises a spindle (15) transforming rotatory movements of the motor (6) into linear movements, preferably, that the rocking mechanism (5) comprises a sled (16) connected between the spindle (15) and the platform (4).

9. Rocking device according to one of the preceding claims, **characterized in that** the rocking device (1) comprises a heating arrangement for heating and/or cooling the bioreactor (2), preferably, that the heating arrangement comprises a, in particular electrical, first heating zone (17) located on the platform (4), in particular below the bioreactor (2), more preferably, that the heating arrangement comprises a, in particular electrical, second heating zone (20) located on the platform (4), in particular below the bioreactor (2).

10. Rocking device according to claim 9, **characterized in that** the control unit (7) is configured to control the first heating zone (17) and the second heating zone (20) differently, and/or,
that the second heating zone (20) comprises two sub-zones (21) located at two sides of the first heating zone (17), in particular one located towards the first end (9) and one located towards the second end (10), preferably, that the two sub-zones (21) are controlled by the control unit (7) together and/or are located at a distance from each other.

11. Rocking device according to claim 9 or 10, **characterized in that** control unit (7) is configured to detect a property, in particular a position and/or a size, of the bioreactor (2) and control the first heating zone (17) and/or the second heating zone (20) based on the detection, preferably, that the control unit (7) is configured to detect via at least one sensor, in particular the tilt sensor (13) and/or at least one weight sensor, a mixing characteristic of the bioreactor (2) associated with the property of the bioreactor (2).

12. Rocking device according to one of the claims 9 to 11, **characterized in that** the heating arrangement comprises a temperature sensor (22), in particular contact temperature sensor, and that the control unit (7) is configured to control the heating arrangement based on measurements from the temperature sensor (22), preferably, that the control unit (7) is configured to control the heating arrangement using a, in particular closed, control loop with the temperature, in particular contact temperature, as a controlled parameter, in particular during a standstill of the platform (4).

13. Rocking device according to one of the claims 9 to 12, **characterized in that** the heating arrangement comprises a heat-transfer fluid arrangement for heating and/or cooling the bioreactor (2), that the heat-transfer fluid arrangement comprises a heat transfer element (23) located on the platform (4), in particular below the bioreactor (2), and a feed arrangement for feeding the heat-transfer fluid to the heat transfer element (23).

14. Rocking device according to claim 13, **characterized in that** the heat transfer element (23) is a, in particular single-embossed, pillow plate (24).

15. Rocking arrangement comprising a rocking device (1) for a bioreactor (2), wherein the rocking device (1) comprises a base (3) and a platform (4) carried by the base (3), wherein the rocking device (1) comprises a rocking mechanism (5) comprising a motor (6), wherein the rocking arrangement (25) comprises a control arrangement comprising at least one control unit (7), wherein the control unit (7) is configured to cause the motor (6) to rock the platform (4) relative to the base (3) around an axis (8),
wherein the platform (4) comprises a first end (9) and a second end (10) located on opposite sides of the axis (8), wherein the rocking mechanism (5) is configured to tilt the platform (4) around the axis (8) starting from a horizontal position in a first direction (11) to a first maximum angle and in an opposite second direction (12) to a second maximum angle,
**characterized in**
**that** the control unit (7) is configured to perform an asymmetric rocking routine comprising at least one asymmetric rocking motion, and, that the asymmetric rocking motion in the asymmetric rocking program comprises tilting the platform (4) in the first direction (11) to a first angle (α), and, tilting the platform (4) in the second direction (12) to a second angle (β) different from the first angle (α),
preferably, that the control arrangement comprises an internal control unit (7), in particular as the control unit (7), as part of the rocking device (1), and/or, that the control arrangement comprises an external control unit (7).

16. Rocking arrangement according to claim 15, **characterized in that** the external control unit (7) or the rocking device (1) comprises a fluid source, in particular a fluid tank (26), connected to the feed arrangement providing the heat-transfer fluid to the heat transfer element (23), and a heating and/or cooling device for heating and/or cooling the heat-transfer fluid, and, that the external control unit (7) is configured to, in a boost routine, stop the supply of the heat-transfer fluid to the heat transfer element (23), increase or decrease the temperature of the heat-transfer fluid, in particular in the tank, and restart supplying the heat-transfer fluid to the heat-transfer element.

17. Method of controlling a rocking arrangement (25) according to one of the claims 15 or 16.
